# EUROPÄISCHE PATENTSCHRIFT

(11) **EP 0 375 617 B1**
(45) Veröffentlichungstag und Bekanntmachung des Hinweises auf die Patenterteilung: **06.04.1994**
(21) Anmeldenummer: 89810952.5
(22) Anmeldetag: 13.12.1989
(51) Int. Cl.: C07D 239/60

(54) **Verfahren zur Herstellung von Pyrimidinderivaten**
Process for the preparation of pyrimidine derivatives
Procédé pour la préparation des dérivés de pyrimidine

(30) Priorität: 22.12.1988 US 288751
(43) Veröffentlichungstag der Anmeldung: 27.06.1990
(73) Patentinhaber: CIBA-GEIGY AG, 4002 Basel (CH)
(72) Erfinder: Seifert, Gottfried, CH-4312 Magden (CH); Hässig, Robert, Dr., CH-5264 Gipf-Oberfrick (CH)

(56) Entgegenhaltungen:
- EP-A- 0 158 594
- EP-A- 0 244 359

## Beschreibung

Die vorliegende Erfindung betrifft ein Verfahren zur Herstellung von 4,6-Bis-difluormethoxypyrimidinen der Formel I 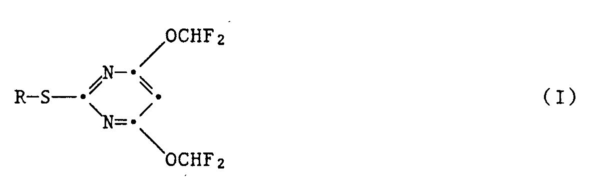
in welcher
R C₁-C₄-Alkyl oder gegebenenfalls substituiertes Phenyl oder
Benzyl bedeutet.

Unter Alkyl ist geradkettiges oder verzweigtes Alkyl zu verstehen, z.B.: Methyl, Aethyl, n-Propyl, i-Propyl, n-Butyl oder die isomeren Butyl.

Als Substituenten für Phenyl oder Benzyl kommen beispielsweise Alkyl, Halogen, Nitro oder Alkoxy in Betracht.

Die 4,6-Bis-difluormethoxypyrimidine der Formel I sind wertvolle Zwischenprodukte. Sie können beispielsweise durch Oxidation in die entsprechenden Sulfone übergeführt werden, die bei der weiteren Umsetzung mit Ammoniak oder einem primären Amin die entsprechenden 2-Amino-4,6-bis-difluormethoxypyrimidine ergeben, die bei der weiteren Umsetzung mit einem geeigneten Phenylsulfonylisocyanat oder N-(Phenylsulfonyl)-carbamat herbizid wirksame Sulfonylharnstoffe liefern. Solche herbizid wirksamen Sulfonylharnstoffe sind beispielsweise in den publizierten europäischen Patentanmeldungen EP-A-0 072 347, EP-A-0 084 020 und EP-A-0 094 790 beschrieben.

Es ist bekannt, 4,6-Bis-difluormethoxy-2-methylthiopyrimidin durch Umsetzung von 4,6-Dihydroxypyrimidin mit Chlordifluormethan in Dioxan in Gegenwart von wässriger Natronlauge herzustellen. Nach diesem Verfahren wird 4,6-Bis-difluormethoxy-2-methylthiopyrimidin lediglich in einer Ausbeute von 25% d. Th. erhalten (vgl. USP 4 542 216, Beispiel 5). Dagegen werden bei der Ueberführung in das 2-Methylsulfonyl-4,6-bis-difluormethoxypyrimidin und dessen Umsetzung zum 2-Amino-4,6-bis-difluormethoxypyrimidin sehr gute bis quantitative Ausbeuten erzielt.

Es ist daher das Ziel der vorliegenden Erfindung, ein Verfahren zur Herstellung der 4,6-Bis-difluormethoxypyrimidine der Formel I bereitzustellen, nach dem diese Verbindungen in guter Ausbeute hergestellt werden können.

Gemäss vorliegender Erfindung wird vorgeschlagen, die 4,6-Bis-difluormethoxypyrimidine der Formel I in der Weise herzustellen, dass man ein 4,6-Dihydroxypyrimidindialkalisalz der Formel II 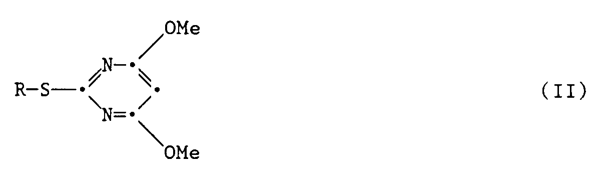
worin
R die unter Formel I angegebene Bedeutung hat und Me für ein Alkalimetall steht, in einem Lösungsmittel aus der Gruppe der Ketone und Alkylcyanide in Gegenwart von 0,05 bis 1,1 Mol Wasser pro Mol eingesetztes Dialkalisalz der Formel II mit Chlordifluormethan umsetzt.

Geeignete Lösungsmittel aus der Gruppe der Ketone sind beispielsweise Aceton, Methyläthylketon, Diäthylketon, Methylisopropylketon oder Methylisobutylketon. Als Lösungsmittel geeignete Alkylcyanide sind beispielsweise Acetonitril oder Propionitril. Bevorzugte Lösungsmittel sind Acetonitril, Propionitril, Aceton und Methyläthylketon. Ein besonders bevorzugtes Lösungsmittel ist Acetonitril. Die vorgenannten Lösungsmittel werden vorteilhaft in einer Menge von 400 bis 3000 ml, vorzugsweise 500 bis 1000 ml pro Mol Dialkalisalz der Formel II verwendet.

Ausgangs- und Endprodukte des erfindungsgemässen Verfahrens sind bekannt. Die Dialkalisalze der Formel II können in an sich bekannter Weise aus den entsprechenden 4,6-Dihydroxypyrimidinen, beispielsweise durch Umsetzung mit Alkalihydroxiden oder Alkalialkoholaten, hergestellt werden.

Besonders vorteilhaft kann die erfindungsgemässe Umsetzung des Dialkalisalzes der Formel II mit Chlordifluormethan in Gegenwart von 0,13 bis 0,6 Mol Wasser pro Mol eingesetztes Dialkalisalz der Formel II durchgeführt werden.

Das erfindungsgemässe Verfahren kann in einem weiten Temperaturbereich durchgeführt werden. Geeignete Reaktionstemperaturen liegen insbesondere im Bereich von + 20°C bis + 100°C. Vorzugsweise wird die Umsetzung bei einer Temperatur von +40°C bis +60°C durchgeführt.

Es ist ferner vorteilhaft, die Umsetzung des Dialkalisalzes der Formel II mit Chlordifluormethan in Gegenwart eines Phasentransferkatalysators durchzuführen. Die Phasentransferkatalysatoren können in einer Menge von 0,01 bis 0,25 Mol pro Mol Dialkalisalz der Formel II eingesetzt werden. Bevorzugt verwendet man 0,05 bis 0,15 Mol Phasentransferkatalysator pro Mol Dialkalisalz der Formel II.

Als Phasentransferkatalysatoren sind generell quartäre Ammoniumsalze und Kronenäther geeignet. Bevorzugte Phasentransferkatalysatoren sind 18-Crown-6, Benzyltrimethylammoniumchlorid, Tetrabutylammoniumchlorid, Tetramethylammoniummethansulfat und Tetramethylammoniumchlorid. Besonders bevorzugt ist Tetramethylammoniumchlorid.

Die Umsetzung des Dialkalisalzes der Formel II mit Chlordifluormethan kann bei Normaldruck oder unter erhöhtem Druck durchgeführt werden. Vorzugsweise wird die Umsetzung unter erhöhtem Druck durchgeführt. Geeignete Drucke liegen im Bereich von 1 bis 100 bar. Ein bevorzugter Druckbereich, in welchem die Reaktion durchgeführt werden kann, liegt bei 1 bis 20 bar.

Das Chlordifluormethan kann in äquimolaren Mengen oder im Ueberschuss eingesetzt werden.

Zweckmässigerweise verwendet man eine Menge von 1,5 bis 10 Mol Chlordifluormethan pro Mol Dialkalisalz der Formel II. Bevorzugt ist eine Menge von 4 bis 6 Mol Chlordifluormethan pro Mol Dialkalisalz der Formel II.

In einer bevorzugten Ausführungsform des erfindungsgemässen Verfahrens wird ein Dinatriumsalz der Formel II in Gegenwart von 0,13 bis 0,6 Mol Wasser und 0,05 bis 0,15 Mol Tetramethylammoniumchlorid pro Mol eingesetztes Dinatriumsalz der Formel II in 500 bis 1000 ml Acetonitril pro Mol Dinatriumsalz der Formel II bei einer Temperatur von +40°C bis +60°C und einem Druck von 1 bis 20 bar mit Chlordifluormethan umgesetzt.

Mit dem erfindungsgemässen Verfahren wird es möglich, die 4,6-Bis-difluormethoxypyrimidine der Formel I ausgehend von den 4,6-Dihydroxypyrimidindialkalisalzen der Formel II in Ausbeuten von bis zu 68 % d. Th. herzustellen, während, wie eingangs erwähnt, nach dem bisher bekannten Verfahren lediglich eine Ausbeute von 25 % d. Th. erzielbar ist.

Ein weiterer Vorteil des erfindungsgemässen Verfahrens besteht darin, dass man das im Ueberschuss eingesetzte Chlordifluormethan für einen neuen Ansatz wiederverwenden kann, während es nach den bekannten Verfahren durch Hydrolyse verloren geht.

Das erfindungsgemässe Verfahren wird durch die folgenden Beispiele näher erläutert:
Beispiel 1: Herstellung von 4,6-Bis-difluormethoxy-2-methylthiopyrimidin
   In einem Rührautoklaven werden 101 g wasserfreies 4,6-Dihydroxy-2-methylthiopyrimidindinatriumsalz und 8 g Tetramethylammoniumchlorid mit 500 ml Acetonitril und 2,5 g Wasser vermischt. Nach Verschliessen des Autoklaven und Aufheizen auf +50°C werden innerhalb von 15 Minuten aus einem Zulaufgefäss aus Stahl 215 g Chlordifluormethan zugegeben, wobei sich im Autoklaven ein Druck von 2 bar einstellt.
   Nach einer Reaktionszeit von 4 Stunden wird der Autoklav belüftet. Anschliessend wird das Reaktionsgemisch abfiltriert und der Filterrückstand mit Acetonitril gewaschen. Nach Abdestillieren des Lösungsmittels im Vakuum bei +80°C und Waschen der Produktschmelze mit 200 ml heissem Wasser erhält man 98 g (68 % d. Th.) 4,6-Bis-difluormethoxy-2-methylthiopyrimidin mit einem Gehalt von 90 %.
Beispiel 2: Wird bei Beispiel 1 das Acetonitril durch Aceton ersetzt, so erhält man bei sonst analoger Arbeitsweise 78 g 4,6-Bis-difluormethoxy-2-methylthiopyrimidin (54 % d. Th.) mit einem Gehalt von 90 %.
Beispiel 3: Wird bei Beispiel 1 das 4,6-Dihydroxy-2-methylthiopyrimidindinatriumsalz durch das entsprechende 4,6-Dihydroxy-2-methylthiopyrimidindikaliumsalz ersetzt, erhält man bei sonst analoger Arbeitsweise 54 g (38 % d. Th.) 4,6-Bis-difluormethoxy-2-methylthiopyrimidin mit einem Gehalt von 90 %.
Beispiel 4: Wird in Beispiel 1 das Tetramethylammoniumchlorid durch Benzyltrimethylammoniumchlorid, Tetrabutylammoniumchlorid oder Tetramethylammoniummethansulfat ersetzt, erhält man bei sonst analoger Arbeitsweise die gleiche Menge an Produkt wie in Beispiel 1.

## Patentansprüche

1. Verfahren zur Herstellung von 4,6-Bis-difluormethoxypyrimidinen der Formel I 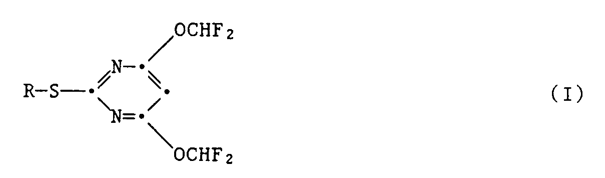 worin
R C₁-C₄-Alkyl oder gegebenenfalls substituiertes Phenyl oder Benzyl bedeutet, dadurch gekenzeichnet, dass man ein 4,6-Dihydroxypyrimidindialkalisalz der Formel II 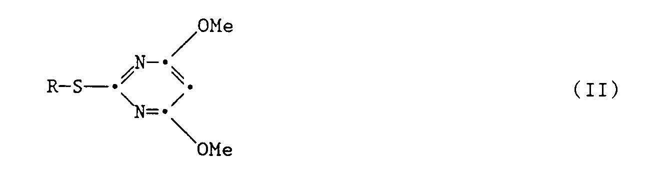 worin
R die unter Formel I angegebene Bedeutung hat und Me für ein Alkalimetall steht, in einem Lösungsmittel aus der Gruppe der Ketone und Alkylcyanide in Gegenwart von 0,05 bis 1,1 Mol Wasser pro Mol eingesetztes Dialkalisalz der Formel II mit Chlordifluormethan umsetzt.

2. Verfahren nach Anspruch 1, dadurch gekennzeichnet, dass man als Lösungsmittel Aceton, Methyläthylketon, Diäthylketon, Methylisopropylketon, Methylisobutylketon, Acetonitril oder Propionitril verwendet.

3. Verfahren nach Anspruch 1, dadurch gekennzeichnet, dass man die Umsetzung des Dialkalisalzes der Formel II mit Chlordifluormethan in Gegenwart von 0,13 bis 0,6 Mol Wasser pro Mol eingesetztes Dialkalisalz der Formel II durchführt.

4. Verfahren nach Anspruch 1, dadurch gekennzeichnet, dass man als Lösungsmittel Acetonitril, Aceton oder Methyläthylketon verwendet.

5. Verfahren nach Anspruch 1, dadurch gekennzeichnet, dass man als Lösungsmittel Acetonitril verwendet.

6. Verfahren nach Anspruch 1, dadurch gekennzeichnet, dass man das Lösungsmittel in einer Menge von 400 bis 3000 ml pro Mol Verbindung der Formel II einsetzt.

7. Verfahren nach Anspruch 1, dadurch gekennzeichnet, dass man das Lösungsmittel in einer Menge von 500 bis 1000 ml pro Mol Verbindung der Formel II einsetzt.

8. Verfahren nach Anspruch 1, dadurch gekennzeichnet, dass Me für Natrium oder Kalium steht.

9. Verfahren nach Anspruch 1, dadurch gekennzeichnet, dass man die Umsetzung des Dialkalisalzes der Formel II mit Chlordifluormethan in Gegenwart eines Phasentransferkatalysators durchführt.

10. Verfahren nach Anspruch 9, dadurch gekennzeichnet, dass man als Phasentransferkatalysator ein quartäres Ammoniumsalz oder einen Kronenäther verwendet.

11. Verfahren nach Anspruch 9, dadurch gekennzeichnet, dass man als Phasentransferkatalysator 18-Crown-6, Benzyltrimethylammoniumchlorid, Tetrabutylammoniumchlorid, Tetramethylammoniummethansulfat oder Tetramethylammoniumchlorid verwendet.

12. Verfahren nach Anspruch 9, dadurch gekennzeichnet, dass man den Phasentransferkatalysator in einer Menge von 0,01 bis 0,25 Mol pro Mol Dialkalisalz der Formel II einsetzt.

13. Verfahren nach Anspruch 9, dadurch gekennzeichnet, dass man den Phasentransferkatalysator in einer Menge von 0,05 bis 0,15 Mol pro Mol Dialkalisalz der Formel II einsetzt.

14. Verfahren nach Anspruch 9 dadurch gekennzeichnet, dass man als Phasentransferkatalysator Tetramethylammoniumchlorid verwendet.

15. Verfahren nach Anspruch 1, dadurch gekennzeichnet, dass man das Dialkalisalz der Formel II bei einer Temperatur von +20°C bis +100°C mit Chlordifluormethan umsetzt.

16. Verfahren nach Anspruch 1, dadurch gekennzeichnet, dass man das Dialkalisalz der Formel II bei einer Temperatur von +40°C bis +60°C mit Chlordifluormethan umsetzt.

17. Verfahren nach Anspruch 1, dadurch gekennzeichnet, dass man das Dialkalisalz der Formel II bei einem Druck von 1 bis 100 bar mit Chlordifluormethan umsetzt.

18. Verfahren nach Anspruch 1, dadurch gekennzeichnet, dass man das Dialkalisalz der Formel II bei einem Druck von 1 bis 20 bar mit Chlordifluormethan umsetzt.

19. Verfahren nach Anspruch 1, dadurch gekennzeichnet, dass Me für Natrium steht.

20. Verfahren nach Anspruch 1, dadurch gekennzeichnet, dass man das Chlordifluormethan in einer Menge von 1,5 bis 10 Mol pro Mol Dialkalisalz der Formel II einsetzt.

21. Verfahren nach Anspruch 1, dadurch gekennzeichnet, dass man das Chlordifluormethan in einer Menge von 4 bis 6 Mol pro Mol Dialkalisalz der Formel II einsetzt.

22. Verfahren nach Anspruch 1, dadurch gekennzeichnet, dass man ein Dinatriumsalz der Formel II in 500 bis 1000 ml Acetonitril pro Mol Dinatriumsalz der Formel II in Gegenwart von 0,13 bis 0,6 Mol Wasser und in Gegenwart von 0,05 bis 0,15 Mol Tetramethylammoniumchlorid pro Mol Dinatriumsalz der Formel II bei einer Temperatur von +40°C bis +60°C und einem Anfangsdruck von 1 bis 20 bar mit Chlordifluormethan unsetzt.

## Claims

1. A process for the preparation of a 4,6-bis(difluoromethoxy)pyrimidine of formula I 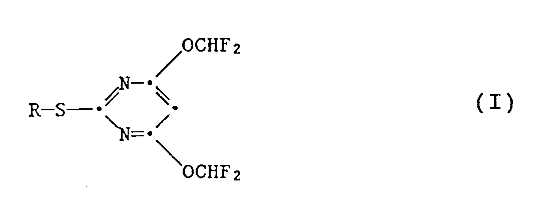 wherein R is C₁-C₄alkyl or unsubstituted or substituted phenyl or benzyl, which process comprises reacting a 4,6-dihydroxypyrimidine dialkali metal salt of formula II 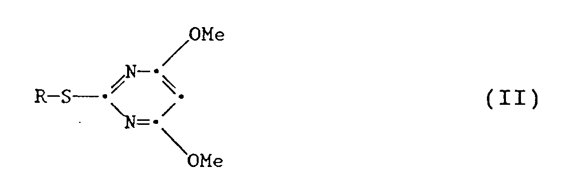 wherein R is as defined for formula I and Me is an alkali metal, with chlorodifluoromethane in a solvent selected from the group consisting of ketones and alkyl cyanides, in the presence of 0.05 to 1.1 mol of water per mol of dialkali metal salt of formula II used.

2. A process according to claim 1, wherein acetone, methyl ethyl ketone, diethyl ketone, methyl isopropyl ketone, methyl isobutyl ketone, acetonitrile or propionitrile is used as the solvent.

3. A process according to claim 1, wherein the reaction of the dialkali metal salt of formula II with chlorodifluoromethane is carried out in the presence of 0.13 to 0.6 mol of water per mol of dialkali metal salt of formula II used.

4. A process according to claim 1, wherein acetonitrile, acetone or methyl ethyl ketone is used as the solvent.

5. A process according to claim 1, wherein acetonitrile is used as the solvent.

6. A process according to claim 1, wherein the solvent is used in an amount of 400 to 3000 ml per mol of compound of formula II.

7. A process according to claim 1, wherein the solvent is used in an amount of 500 to 1000 ml per mol of compound of formula II.

8. A process according to claim 1, wherein Me is sodium or potassium.

9. A process according to claim 1, wherein the reaction of the dialkali metal salt of formula II with chlorodifluoromethane is carried out in the presence of a phase transfer catalyst.

10. A process according to claim 9, wherein a quaternary ammonium salt or a crown ether is used as the phase transfer catalyst.

11. A process according to claim 9, wherein 18-crown-6, benzyltrimethylammonium chloride, tetrabutylammonium chloride, tetramethylammonium methyl sulfate or tetramethylammonium chloride is used as the phase transfer catalyst.

12. A process according to claim 9, wherein the phase transfer catalyst is used in an amount of 0.01 to 0.25 mol per mol of dialkali metal salt of formula II.

13. A process according to claim 9, wherein the phase transfer catalyst is used in an amount of 0.05 to 0.15 mol per mol of dialkali metal salt of formula II.

14. A process according to claim 9, wherein tetramethylammonium chloride is used as the phase transfer catalyst.

15. A process according to claim 1, wherein the dialkali metal salt of formula II is reacted with chlorodifluoromethane at a temperature of from +20°C to +100°C.

16. A process according to claim 1, wherein the dialkali metal salt of formula II is reacted with chlorodifluoromethane at a temperature of from +40°C to +60°C.

17. A process according to claim 1, wherein the dialkali metal salt of formula II is reacted with chlorodifluoromethane under a pressure of from 1 to 100 bar.

18. A process according to claim 1, wherein the dialkali metal salt of formula II is reacted with chlorodifluoromethane under a pressure of from 1 to 20 bar.

19. A process according to claim 1, wherein Me is sodium.

20. A process according to claim 1, wherein chlorodifluoromethane is used in an amount of 1.5 to 10 mol per mol of dialkali metal salt of formula II.

21. A process according to claim 1, wherein chlorodifluoromethane is used in an amount of 4 to 6 mol per mol of dialkali metal salt of formula II.

22. A process according to claim 1, which comprises reacting a disodium salt of formula II with chlorodifluoromethane in the presence of 0.13 to 0.6 mol of water and 0.05 to 0.15 mol of tetramethylammonium chloride per mol of disodium salt of formula II, in 500 to 1000 ml of acetonitrile per mol of disodium salt of formula II, at a temperature of from +40°C to +60°C and under an initial pressure of from 1 to 20 bar.

## Revendications

1. Procédé pour la préparation des 4,6-bis-difluorométhoxypyrimidines de formule I 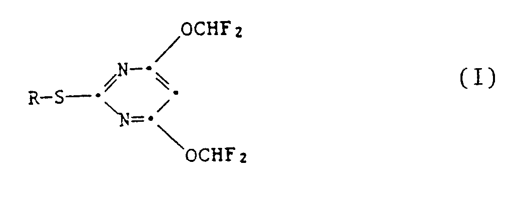 où
R représente un alkyle en C₁-C₄ ou le phényle ou le benzyle éventuellement substitué, caractérisé en ce que l'on fait réagir un sel dialcalin de 4,6-dihydroxypyrimidine de formule II 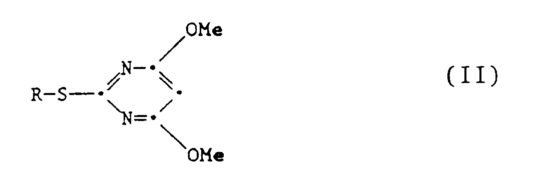 où
R a la signification donnée pour la formule I et Me représente un métal alcalin, dans un solvant du groupe des cétones et des cyanures d'alkyle, en présence de 0,05 à 1,1 mole d'eau par mole de sel dialcalin de formule II utilisé avec le chlorodifluorométhane.

2. Procédé selon la revendication 1, caractérisé en ce que l'on utilise, comme solvant, l'acétone, la méthyléthylcétone, la diéthylcétone, la méthylisopropylcétone, la méthylisobutylcétone, l'acétonitrile ou le propionitrile.

3. Procédé selon la revendication 1, caractérisé en ce que l'on fait réagir le sel dialcalin de formule II avec le chlorodifluorométhane, en présence de 0,13 à 0,6 mole d'eau par mole de sel dialcalin de formule II utilisé.

4. Procédé selon la revendication 1, caractérisé en ce que l'on utilise, comme solvant, l'acétonitrile, l'acétone ou la méthyléthylcétone.

5. Procédé selon la revendication 1, caractérisé en ce que l'on utilise, comme solvant, l'acétonitrile.

6. Procédé selon la revendication 1, caractérisé en ce que l'on utilise le solvant en une quantité allant de 400 à 3 000 ml par mole de composé de formule II.

7. Procédé selon la revendication 1, caractérisé en ce que l'on utilise le solvant en une quantité allant de 500 à 1 000 ml par mole de composé de formule II.

8. Procédé selon la revendication 1, caractérisé en ce que Me représente le sodium ou le potassium.

9. Procédé selon la revendication 1, caractérisé en ce que l'on fait réagir le sel dialcalin de formule II avec le chlorodifluorométhane, en présence d'un catalyseur de transfert de phases.

10. Procédé selon la revendication 9, caractérisé en ce que l'on utilise, comme catalyseur de transfert de phases, un sel d'ammonium quaternaire ou un éther-couronne.

11. Procédé selon la revendication 9, caractérisé en ce que l'on utilise, comme catalyseur de transfert de phases, le 18-couronne-6, le chlorure de benzyltriméthylammonium, le chlorure de tétrabutylammonium, le méthanesulfate de tétraméthylammonium ou le chlorure de tétraméthylammonium.

12. Procédé selon la revendication 9, caractérisé en ce que l'on utilise le catalyseur de transfert de phases en une quantité allant de 0,01 à 0,25 mole de sel dialcalin de formule II.

13. Procédé selon la revendication 9, caractérisé en ce que l'on utilise le catalyseur de transfert de phases en une quantité allant de 0,05 à 0,15 mole par mole de sel dialcalin de formule II.

14. Procédé selon la revendication 9, caractérisé en ce que l'on utilise, comme catalyseur de transfert de phases, le chlorure de tétraméthylammonium.

15. Procédé selon la revendication 1, caractérisé en ce que l'on fait réagir le sel dialcalin de formule II à une température allant de +20°C à +100°C avec le chlorodifluorométhane.

16. Procédé selon la revendication 1, caractérisé en ce que l'on fait réagir le sel dialcalin de formule II à une température allant de +40°C à +60°C avec le chlorodifluorométhane.

17. Procédé selon la revendication 1, caractérisé en ce que l'on fait réagir le sel dialcalin de formule II sous une pression allant de 1 à 100 bars avec le chlorodifluorométhane.

18. Procédé selon la revendication 1, caractérisé en ce que l'on fait réagir le sel dialcalin de formule II sous une pression allant de 1 à 20 bars avec le chlorodifluorométhane.

19. Procédé selon la revendication 1, caractérisé en ce que Me représente le sodium.

20. Procédé selon la revendication 1, caractérisé en ce que l'on utilise le chlorodifluorométhane en une quantité allant de 1,5 à 10 moles par mole de sel dialcalin de formule II.

21. Procédé selon la revendication 1, caractérisé en ce que l'on utilise le chlorodifluorométhane en une quantité allant de 4 à 6 modes par mode de sel dialcalin de formule II.

22. Procédé selon la revendication 1, caractérisé en ce que l'on fait réagir un sel disodique de formule II dans 500 à 1 000 ml d'acétonitrile par mole de sel disodique de formule II, en présence de 0,13 à 0,6 mode d'eau et en présence de 0,05 à 0,15 mode de chlorure de tétraméthylammonium par mode de sel disodique de formule II à une température allant de +40°C à +60°C et sous une pression de départ allant de 1 à 20 bars avec de chlorodifluorométhane.
